(19) Europäisches Patentamt / European Patent Office / Office européen des brevets

(11) EP 4 209 525 A1

(12) # EUROPEAN PATENT APPLICATION
published in accordance with Art. 153(4) EPC

(43) Date of publication:
**12.07.2023 Bulletin 2023/28**

(21) Application number: **21864622.2**

(22) Date of filing: **31.08.2021**

(51) International Patent Classification (IPC):
***C08G 18/38*** (2006.01) ***C08G 18/70*** (2006.01)
***C07C 319/02*** (2006.01) ***C08L 75/04*** (2006.01)
***G02B 1/04*** (2006.01) ***C07C 319/20*** (2006.01)
***C07C 319/28*** (2006.01) ***C07C 321/14*** (2006.01)
***C07C 321/04*** (2006.01)

(52) Cooperative Patent Classification (CPC):
**C07C 319/02; C07C 319/20; C07C 319/28; C07C 321/04; C07C 321/14; C08G 18/38; C08G 18/70; C08L 75/04; G02B 1/04**

(86) International application number:
**PCT/KR2021/011694**

(87) International publication number:
**WO 2022/050662 (10.03.2022 Gazette 2022/10)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **01.09.2020 KR 20200111005**
**01.12.2020 KR 20200165577**

(71) Applicant: **SKC Co., Ltd.**
**Suwon-si, Gyeonggi-do 16336 (KR)**

(72) Inventors:
- **PAI, Jae Young**
  **Suwon-si, Gyeonggi-do 16338 (KR)**
- **YOU, Kyeong Hwan**
  **Suwon-si, Gyeonggi-do 16338 (KR)**
- **MYUNG, Jung Hwan**
  **Suwon-si, Gyeonggi-do 16338 (KR)**
- **HAN, Hyuk Hee**
  **Suwon-si, Gyeonggi-do 16338 (KR)**
- **KIM, Jeong Moo**
  **Suwon-si, Gyeonggi-do 16338 (KR)**
- **CHOI, Eui Jun**
  **Suwon-si, Gyeonggi-do 16338 (KR)**
- **SHIN, Jung Hwan**
  **Suwon-si, Gyeonggi-do 16338 (KR)**

(74) Representative: **Stolmár & Partner Patentanwälte PartG mbB**
**Blumenstraße 17**
**80331 München (DE)**

(54) **POLYTHIOL COMPOSITION, OPTICAL COMPOSITION, AND OPTICAL PRODUCTS**

(57) A polythiol composition according to exemplary examples includes a tetrafunctional polythiol compound, and a sub-polythiol compound including a compound represented by $C_{13}H_{28}S_9$ and a compound represented by $C_{15}H_{32}S_{10}$. A ratio of the sub-polythiol compound measured through a high performance liquid chromatography (HPLC) analysis graph is 1% to 5%. By adjusting the content of the sub-polythiol compound, it is possible to manufacture an optical product having excellent transmittance and optical properties.

EP 4 209 525 A1

## Description

CROSS-REFERENCE TO RELATED APPLICATION

This application claims priority to Korean Patent Application No. 10-2020-0111005 filed on September 01, 2020 and Korean Patent Application No. 10-2020-0165577 filed on December 01, 2020 in the Korean Intellectual Property Office (KIPO), the entire disclosure of which is incorporated by reference herein.

BACKGROUND

1. Field

The present invention relates to a polythiol composition, an optical composition and an optical product. More particularly, the present inventions relates to a polythiol composition including a plurality of polythiol-based compounds, and an optical composition and an optical product including the same.

2. Description of the Related Art

A polythiol compound is widely used, for example, as a raw material for manufacturing a polyurethane resin. For example, a polythiol compound is used to manufacture an optical lens using a polyurethane resin, and quality such as purity of the polythiol compound as a raw material may directly affect the quality of the optical lens.
For example, a polythiourethane-based compound prepared by reacting a polythiol compound and an isocyanate compound may be used as a base material of the optical lens.
For example, Korean Patent Laid-Open Publication No. 10-1338568 discloses a method for synthesizing a polythiol compound by reacting a polyol compound with thiourea to prepare an isothiouronium salt, and then hydrolyzing it using aqueous ammonia.
During the synthesizing process, a reaction additive such as a catalyst may be introduced and, depending on physical properties of the reaction additive, the purity, yield, etc. of the synthesized polythiol compound may be varied. Further, depending on the number of functional groups, a chain length, molecular weight, purity, etc. of the synthesized polythiol, optical properties of a lens such as a transparency, a refractive index, etc. may be minutely changed.
Accordingly, optical properties such as the refractive index and color of an optical product such as an optical lens prepared from a polythiol compound may also be changed.

SUMMARY

An object according to exemplary embodiments is to provide a polythiol composition having improved transparency and optical properties, and a method for preparing the same.
In addition, another object according to exemplary embodiments is to provide an optical composition including a polythiol composition having improved transparency and optical properties, and a method for preparation thereof.
Further, another object according to exemplary embodiments is to provide an optical product manufactured using the polythiol composition or the optical composition.
Furthermore, another object according to exemplary embodiments is to provide a metal sulfide for synthesizing a polythiol-based compound.
A polythiol composition according to exemplary embodiments includes: a tetrafunctional polythiol compound; and a sub-polythiol compound including a compound represented by $C_{13}H_{28}S_9$ and a compound represented by $C_{15}H_{32}S_{10}$, wherein a ratio of the sub-polythiol compound represented by Equation 1 below ranges from 1% to 5%:

[Equation 1]

$$\text{Sub-polythiol compound ratio} = 100\% \times [(\text{Peak region (\%) of } C_{13}H_{28}S_9) + (\text{Peak region (\%) of } C_{15}H_{32}S_{10})]/(\text{Peak region of tetrafunctional polythiol compound (\%)})$$

(In Equation 1, the peak region (%) is a peak area (%) of the compound measured through a high performance liquid chromatography (HPLC) analysis graph obtained at a wavelength of 230 nm).
In some embodiments, the tetrafunctional polythiol compound may include at least one of tetrafunctional polythiol compounds represented by Formulae 1-1 to 1-3 below:

[Formula 1-1]

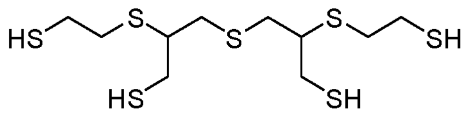

[Formula 1-2]

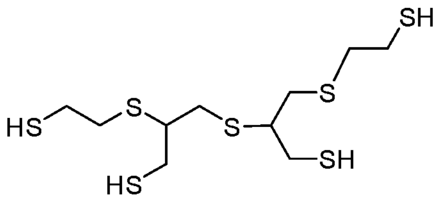

[Formula 1-3]

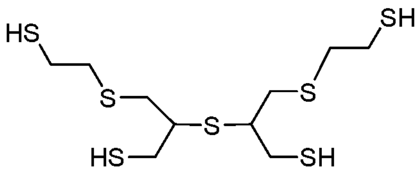

In some embodiments, the compound represented by $C_{13}H_{28}S_9$ may have a structure of Formula 2-1 below:

[Formula 2-1]

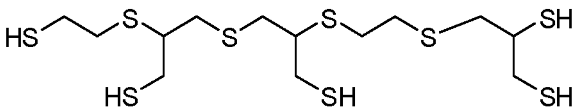

In some embodiments, the compound represented by $C_{15}H_{32}S_{10}$ may have a structure of Formula 2-2 below:

[Formula 2-2]

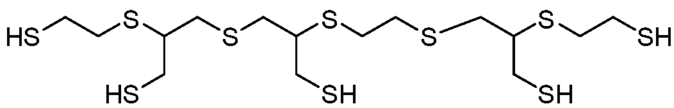

A method for preparation of a polythiol composition according to exemplary embodiments includes: generating a polyol intermediate by introducing a metal sulfide to a preliminary polyol compound; and converting the polyol intermediate into a polythiol-based compound through thiolation. The metal sulfide has an absorbance of 0.7 to 2.0 when measured for light at a wavelength of 350 nm in a quartz cell having an optical path length of 50 mm after dissolving the metal sulfide in distilled water in an amount of 17.3 parts by weight based on 100 parts by weight of distilled water.

In some embodiments, the polythiol-based compound may include a tetrafunctional polythiol compound, and a sub-polythiol compound having a greater molecular weight or a greater functional number than the tetrafunctional polythiol compound.

In some embodiments, the sub-polythiol compound may include a compound represented by $C_{13}H_{28}S_9$ and a compound represented by $C_{15}H_{32}S_{10}$, and a ratio of the sub-polythiol compound represented by the above Equation 1 may range from 1% to 5%.

In some embodiments, the method may include, if the absorbance of the metal sulfide is less than 0.7, washing the metal sulfide with alcohol, water or an aqueous alcohol solution and then drying the same to adjust the absorbance of the metal sulfide in a range of 0.7 to 2.0

In some embodiments, the metal sulfide may include $Na_2S$.

In some embodiments, the absorbance of the metal sulfide may range from 0.75 to 2.0.

An optical composition according to exemplary embodiments includes: a polythiol composition which comprises a tetrafunctional polythiol compound, and a sub-polythiol compound including a compound represented by $C_{13}H_{28}S_9$ and a compound represented by $C_{15}H_{32}S_{10}$, wherein a ratio of the sub-polythiol compound represented by the above Equation 1 ranges from 1% to 5%; and an isocyanate-based compound.

A method for preparation of an optical composition according to exemplary embodiments includes: preparing a polythiol-based compound; and admixing the polythiol-based compound with an isocyanate-based compound. The step of preparing the polythiol-based compound includes: generating a polyol intermediate by introducing a metal sulfide to a preliminary polyol compound; and converting the polyol intermediate into a polythiol-based compound through thiolation. The metal sulfide has an absorbance of 0.7 to 2.0 when measured for light at a wavelength of 350 nm in a quartz cell having an optical path length of 50 mm after dissolving the metal sulfide in distilled water in an amount of 17.3 parts by weight based on 100 parts by weight of distilled water.

An optical product according to exemplary embodiments includes a copolymer of a polythiol composition and an isocyanate-based compound, wherein the polythiol composition comprises a tetrafunctional polythiol compound, and a sub-polythiol compound including a compound represented by $C_{13}H_{28}S_9$ and a compound represented by $C_{15}H_{32}S_{10}$, and wherein a ratio of the sub-polythiol compound represented by the above Equation 1 ranges from 1% to 5%:

In some embodiments, the optical product may further include at least one additive selected from the group consisting of a release agent, a reaction catalyst, a thermal stabilizer, an ultraviolet absorber and a bluing agent.

A metal sulfide for synthesizing a polythiol-based compound according to exemplary embodiments has an absorbance of 0.7 to 2.0 when measured for light at a wavelength of 350 nm in a quartz cell having an optical path length of 50 mm after dissolving the same in distilled water in an amount of 17.3 parts by weight based on 100 parts by weight of distilled water.

According to the above-described embodiments, the polythiol composition according to the exemplary embodiments may include a tetrafunctional polythiol-based compound, and a sub-polythiol compound having a predetermined structure and included in a predetermined content range measured by HPLC.

The sub-polythiol compound may have a relatively large chain length or a large functional number, that is, a large number of functional groups. By adjusting the content of the sub-polythiol compound, it is possible to improve the yield and purity of a lens while reducing optical defects of the polyol composition such as white turbidity, yellowing, stria phenomena and the like.

In some embodiments, a metal sulfide having an absorbance in a predetermined range to a light at a wavelength of 350 nm may be used during synthesis of the polythiol-based compound. By controlling the absorbance of the metal sulfide, the content of the sub-polythiol compound may be finely adjusted. Further, the excessive use of the metal sulfide for formation of a polyol intermediate is prevented, thereby inhibiting the excessive formation of the polymer material and the sulfide bond.

Accordingly, it is possible to obtain a polythiol-based compound or polythiol composition capable of implementing an optical product having excellent optical properties along with high purity, while inhibiting discoloration, white turbidity, etc.

## DETAILED DESCRIPTION OF THE EMBODIMENTS

Hereinafter, embodiments of the present application will be described in detail. In this regard, the present invention may be altered in various ways and have various embodiments, such that specific embodiments will be illustrated in the drawings and described in detail in the present disclosure. However, the present invention is not limited to the specific embodiments, and it will be understood by those skilled in the art that the present invention is to cover all modifications, equivalents, and alternatives falling within the spirit and scope of the present invention.

Unless otherwise defined, all terms including technical and scientific terms used herein have the same meaning as commonly understood by one of ordinary skill in the art to which the present invention belongs. It will be further understood that terms, such as those defined in commonly used dictionaries, should be interpreted as having a meaning that is consistent with their meaning in the context of the relevant art and will not be interpreted in an idealized or overly formal sense unless expressly so defined herein.

### <Polythiol composition and method for preparation thereof>

According to one aspect of the present application, there is provided a polythiol composition which includes polythiol-based compounds.

According to exemplary embodiments, the polythiol composition may include a tetrafunctional polythiol compound and a sub-polythiol compound.

The tetrafunctional polythiol compound may be included as a target polythiol compound or a main polythiol compound of the polythiol composition. For example, the tetrafunctional polythiol compound has a relatively smaller molecular weight or shorter molecular length than the sub-polythiol compound to be described below, and may provide sufficient reaction rate/reaction efficiency during polymerization with an isocyanate-based compound for manufacturing a lens.

Non-limiting examples of the tetrafunctional polythiol compound may include compounds represented by Formulae 1-1 to 1-3 below. For example, the main polythiol compound may include at least one among the compounds represented by Formula 1-1 to Formula 1-3 below.

[Formula 1-1]

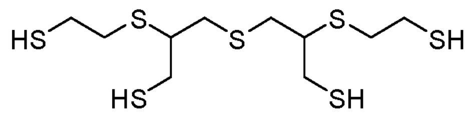

[Formula 1-2]

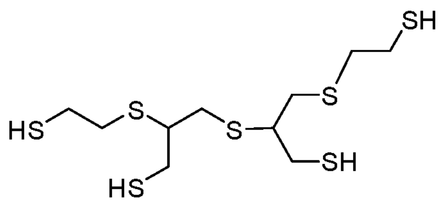

[Formula 1-3]

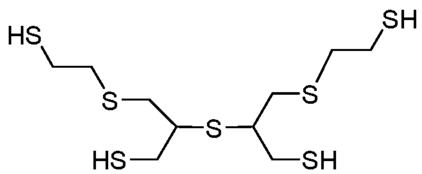

In a preferred embodiment, the compound represented by Formula 1-1 may be used as the tetrafunctional polythiol compound.

The sub-polythiol compound may have a greater chain length, greater molecular weight, or greater functional number (e.g., the number of thiol functional groups) than the tetrafunctional polythiol compound.

As the sub-polythiol compound has a molecular weight or chain length greater than that of the tetrafunctional polythiol compound, it may serve as a reaction rate regulator. For example, because of the sub-polythiol compound, the generation of stria due to an excessively fast reaction rate with an isocyanate-based compound may be reduced or suppressed during a lens manufacturing process to be described below.

In some embodiments, the sub-polythiol compound may be a pentafunctional polythiol compound. In one embodiment, the sub-polythiol compound may include at least two different pentafunctional polythiol compounds.

In some embodiments, the sub-polythiol compound may include a compound represented by $C_{13}H_{28}S_9$. For example, the sub-polythiol compound may include a compound represented by Formula 2-1 below.

[Formula 2-1]

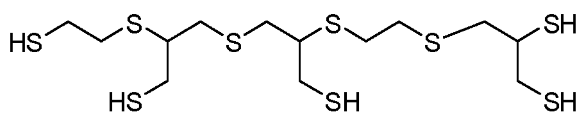

In some embodiments, the sub-polythiol compound may include a compound represented by $C_{15}H_{32}S_{10}$. For example, the sub-polythiol compound may include a compound represented by Formula 2-2 below.

[Formula 2-2]

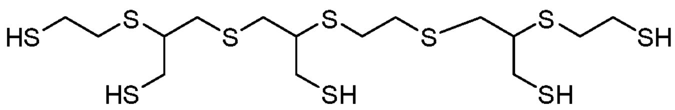

According to exemplary embodiments, a ratio of the sub-polythiol compound defined by Equation 1 below may be in a range of 1 to 5%.

[Equation 1]

$$\text{Sub-polythiol compound ratio} = 100\% \times [(\text{Peak region (\%) of } C_{13}H_{28}S_9) + (\text{Peak region (\%) of } C_{15}H_{32}S_{10})]/(\text{Peak region of tetrafunctional polythiol compound (\%)})$$

The peak region (%) used in Equation 1 is a peak area (%) of the compound measured through a high performance liquid chromatography (HPLC) analysis graph obtained at a wavelength of 230 nm.

In the sub-polythiol compound ratio range represented by Equation 1 above, the chemical stability of the polythiol composition or the optical product may be improved, and thus white turbidity and color shift of the lens may be effectively inhibited.

For example, within the above range of the sub-polythiol compound ratio, an amount of high molecular weight components in the polythiol composition is appropriately controlled while maintaining an appropriate reaction rate with the isocyanate-based compound, and a generation of stria in the optical product and a reduction in purity may also be inhibited.

Further, during the synthesizing process of the polythiol compound to be described below, the generation of poly-sulfide may be appropriately suppressed thus to inhibit color shift such as yellowing of the lens.

In a preferred embodiment, a ratio of the sub-polythiol compound defined by Formula 1 may be in a range of 2% to 5%, 3% to 5%, or 3.5% to 4.8%.

In some embodiments, the polythiol composition has a thiol value (SHV) of about 95 g/eq to 97 g/eq. Preferably, the SHV is 96 g/eq to 97 g/eq., and more preferably 96 g/eq to 96.5 g/eq.

SHV may be measured as a value obtained by dividing the sample weight by the consumed iodine equivalent when titrating a polythiol composition sample using a 0.1N iodine standard solution.

In some embodiments, a liquid refractive index of the polythiol composition may be about 1.645 to 1.647. Preferably, the liquid refractive index may be about 1.6455 to 1.6468, and more preferably 1.6458 to 1.6468.

The liquid refractive index may be measured at 25 °C using a liquid refractometer.

In some embodiments, a gel permeation chromatography (GPC) purity of the polythiol composition may be 80% or more. For example, the GPC of the polythiol composition may be 80% to 85%. Preferably, the GPC purity is 82% or more, more preferably 83% or more, and still more preferably 84% or more.

According to one aspect of the present application, there is provided a method for preparing a polythiol composition including polythiol-based compounds. As described above, the polythiol composition may include a tetrafunctional polythiol compound and the above-described sub-polythiol compound.

A method for preparing a polythiol composition according to exemplary embodiments may include at least one of the steps, processes or actions described as S10, S20 and S30 below. It should be understood that the following terms "S10" and "S20" are used to distinguish processes for the convenience of description and are not intended to limit the sequential order thereof. For example, some or all of the processes of S10, S20 and S30 below may be sequentially conducted, and/or may be conducted with altered order according to process conditions.

S10) Introducing a metal sulfide to a preliminary polyol compound to produce a polyol intermediate.
S20) Reacting the polyol intermediate with thiourea under acidic conditions to produce an isothiouronium salt
S30) Converting the isothiouronium salt into a polythiol-based compound

For example, in step S10, the polyol intermediate may be produced by reacting the preliminary polyol compound with the metal sulfide.

In one embodiment, the preliminary polyol compound may be obtained through a reaction with 2-mercaptoethanol and epihalohydrin as exemplified in Scheme 1 below.

[Scheme 1]

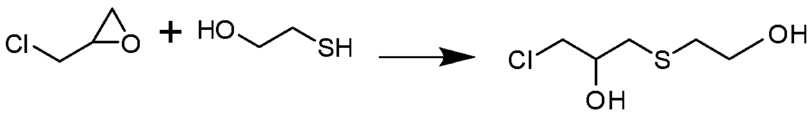

A basic catalyst may be used to promote the reaction of 2-mercaptoethanol and epihalohydrin. Examples of the basic catalyst may include tertiary amines such as triethyl amine, quaternary ammonium salts, triphenylphosphine, and trivalent chromium-based compounds. Epichlorohydrin may be used as epihalohydrin as illustrated in Scheme 1.

The obtained preliminary polyol compound may be, for example, a diol compound containing a sulfide bond.

A reaction temperature for generating the preliminary polyol compound may be, for example, -5 °C to 15 °C, preferably 0 °C to 12 °C, and more preferably 5 °C to 10 °C.

For example, a content of 2-mercaptoethanol may be 0.5 moles to 3 moles, preferably 0.7 moles to 2 moles, and more preferably 0.9 moles to 1.1 moles, based on 1 mole of epihalohydrin. The basic catalyst may be used in an amount of 0.001 moles to 0.1 moles based on 1 mole of epihalohydrin.

In one embodiment, a metallic catalyst such as sodium hydroxide and potassium hydroxide as a reaction catalyst for generating the preliminary polyol compound may be excluded to suppress by-products such as trifunctional thiol.

By introducing a metal sulfide to the diol compound having a sulfide bond obtained as described above, a polyol intermediate may be formed, as illustrated in Scheme 2 below.

[Scheme 2]

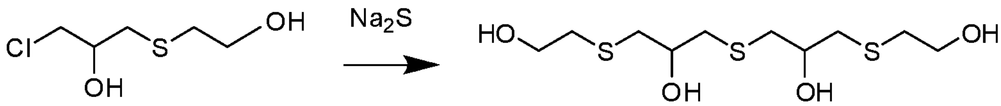


As illustrated in Scheme 2, the diol compounds may be further reacted with each other through the metal sulfide to obtain a polyol intermediate including a tetrafunctional polyol compound.

The metal sulfide may include alkali metal sulfide, and in one embodiment, as shown in Scheme 2, $Na_2S$ may be used.

According to exemplary embodiments, a ratio of the above-described sub-polythiol compound may be controlled by adjusting the absorbance of metal sulfide or $Na_2S$.

In some embodiments, the metal sulfide used herein may be one having an absorbance of 0.7 to 2.0 when the absorbance is measured for light at a wavelength of 350 nm at 25 °C after dissolving the same in distilled water in an amount of 17.3 parts by weight ("wt. parts") based on 100 wt. parts of distilled water in a 50 mm quartz cell.

In a preferred embodiment, the absorbance of the metal sulfide may be 0.75 to 2.0, and preferably 0.75 to 1.95 or 0.75 to 1.5.

Within the above absorbance range, the metal sulfide may be used in a state in which an appropriate amount of moisture is included. Therefore, a metal sulfide corresponding to a sufficient reaction equivalent may be provided through a predetermined dosage.

The metal sulfide may act as a base to promote the reaction of the diol compound exemplified by Scheme 2 above. Accordingly, the polyol intermediate may be easily obtained at a desired reaction rate and production amount.

For example, the metal sulfide may be bound with a leaving group generated during the reaction of Scheme 2 through an ionic bond, a metal bond or a covalent bond, and thus may mediate conversion into the polyol intermediate.

**According** to exemplary embodiments, the metal sulfide in the absorbance range may be used to promote the production of the polyol intermediate with high efficiency.

For example, when a sufficient reaction equivalent of $Na_2S$ is not supplied, high molecular weight by-products such as oligomers may be generated, and a purity of the synthesized polythiol-based compound may be reduced. Accordingly, the amount of the above-described sub-polythiol compound may be excessively increased, and a proportion (or ratio) of the sub-polythiol compound represented by Formula 1 may exceed 5%.

Further, when a metal sulfide having an excessively high absorbance is used, polysulfide compound production may excessively occur to cause discoloration and yellowing of the optical product.

Accordingly, it is possible to provide a polythiol composition that can suppress the formation of high molecular weight by-products by employing a metal compound having an absorbance in the above range and may provide an optical product with high transparency.

In a preferred embodiment, the absorbance of the metal compound may be 0.75 to 2.0, and preferably 0.75 to 1.95 or 0.75 to 1.5.

When a commercially purchased metal sulfide is used, a product in the above-described absorbance range may be selected and used. Alternatively, it may be further processed to have the above-described absorbance range.

In one embodiment, when a reaction equivalent corresponding to the number of moles of reaction sufficient for the conversion reaction of the above-described diol compound into a tetrafunctional polyol compound is not provided or when a metal sulfide product with excessively low absorbance is purchased, it may be sufficiently washed with a cleaning solution on a filter, followed by drying the same. Then, after re-dissolving 17.3 wt. parts of the product in 100 wt. parts of distilled water, an absorbance of the solution to the 350 nm wavelength light is measured in a 50 mm quartz cell to confirm that the absorbance in the range of 0.7 to 2.0 is obtained. Thereafter, the metal sulfide may be used.

For example, as the cleaning solution, alcohol, water or an aqueous alcohol solution of 10 °C or lower may be used. For example, ethanol may be used as the alcohol.

In one embodiment, when a metal sulfide product having an excessively high absorbance is purchased, it is uniformly admixed with a metal sulfide product having an absorbance of less than 2.0. Then, after confirming that an absorbance in a range of 0.7 to 2.0 is obtained according to the absorbance measurement method described above, the mixture may be used.

For example, in step S20, the polyol intermediate may be reacted with thiourea. As a result, according to exemplary embodiments, an isothiouronium salt may be obtained.

Acidic condition reflux may be used in the production of isothiouronium salts. In order to form the acidic conditions, acidic compounds such as hydrochloric acid, hydrobromic acid, iodic acid, sulfuric acid, phosphoric acid, and the like may be used.

The reflux temperature may be 80 °C to 150 °C, 90 °C to 130 °C, and preferably 100 °C to 120 °C, while the reflux time may be 1 hours to 10 hours, 2 hours to 8 hours, 2 hours to 5 hours, and preferably 3 hours to 5 hours.

For example, in step S30, the isothiouronium salt may be converted into a polythiol-based compound. According to exemplary embodiments, the isothiouronium salt may be hydrolyzed under basic conditions to produce a polythiol-based compound.

Steps S20 and S30 described above may include thiolation exemplified by Scheme 3 below.

[Scheme 3]

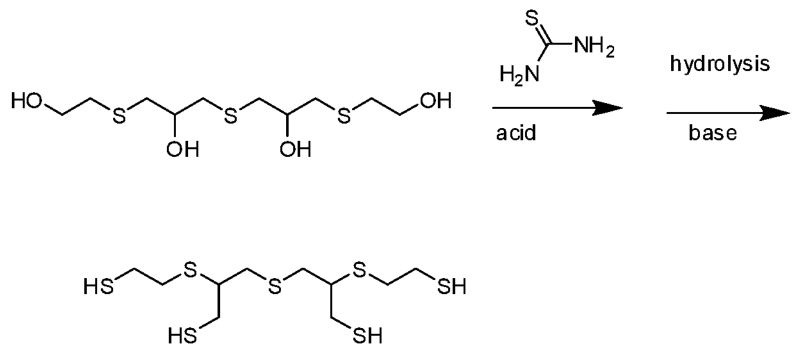


For example, it may be hydrolyzed by adding a basic aqueous solution to the reaction solution containing the isothiouronium salt. The basic aqueous solution may include alkali metal hydroxide, alkaline earth metal hydroxide and/or alkali metal hydride, such as NaOH, KOH, LiOH, $Ca(OH)_2$, etc.

In one embodiment, the reaction solution containing the isothiouronium salt is cooled to a temperature of 20 °C to 100 °C, preferably 20°C to 80 °C, and more preferably 30 °C to 70 °C or 40°C to 70 °C. Thereafter, the basic aqueous solution may be added.

In one embodiment, an organic solvent may be added before adding the basic aqueous solution. An organic solvent having low reactivity or substantially no reactivity and a boiling point exceeding a thiolation reaction temperature may be used so as to allow a thiolation reaction to proceed stably.

Examples of the organic solvent may include toluene, xylene, chlorobenzene, and dichlorobenzene. Preferably, toluene may be used in consideration of reaction stability and toxicity from an organic solvent.

The polythiol-based compound obtained as described above may be further purified. For example, by repeatedly performing acid washing and water washing processes, impurities included in the polythiol-based compound may be removed, in addition, the transparency of the optical material prepared from the polythiol composition may be improved. Thereafter, drying, filtration, etc. may be additionally performed.

In one embodiment, an aqueous layer may be separated or removed through layer separation after proceeding

with the hydrolysis. Acid washing may be carried out at a temperature of about 20°C to 50 °C, and preferably about 30°C to 40 °C for 20 minutes to 1 hour, or 20 minutes to 40 minutes by introducing an acid solution to the obtained organic phase solution.

After the acid washing, a water washing process may be conducted by adding degassed water having a dissolved oxygen concentration adjusted to 5 ppm or less, preferably 3 ppm or less, and more preferably 2 ppm or less. The water washing process may be conducted at a temperature of about 20 °C to 50 °C, preferably about 35°C to 45 °C for 20 minutes to 1 hour, or 20 minutes to 40 minutes. The water washing process may be repeated two or more times, for example, may be conducted 3 to 6 times.

After the acid washing and water washing process, the residual organic solvent and moisture are removed by heating under reduced pressure, followed by filtering through a filter to obtain a polythiol-based compound with high purity.

<Optical composition and optical product>

According to one aspect of the present application, there is provided an optical composition including the above-described polythiol-based compound or polythiol composition. The optical composition may be a polymerizable composition for an optical material for manufacturing an optical product such as a lens.

The optical composition may include the polythiol-based compound or polythiol composition, as well as an isocyanate-based compound.

The isocyanate-based compound may include a compound that is useable as a monomer for synthesizing polythiourethane. In a preferred embodiment, the isocyanate-based compound may include 1,3-bis(isocyanatomethyl)cyclohexane, hexamethylene diisocyanate, isophorone diisocyanate, xylene diisocyanate, toluene diisocyanate and the like. These may be used alone or in combination of two or more thereof.

The optical composition may further include additives such as a release agent, a reaction catalyst, a thermal stabilizer, an ultraviolet absorber, a bluing agent and the like.

Examples of the release agent may include a fluorine-based nonionic surfactant having a perfluoroalkyl group, a hydroxyalkyl group or a phosphoric acid ester group; a silicone-based nonionic surfactant having a dimethylpolysiloxane group, a hydroxyalkyl group or a phosphoric acid ester group; alkyl quaternary ammonium salts such as trimethylcetyl ammonium salt, trimethylstearyl, dimethylethylcetyl ammonium salt, triethyldodecyl ammonium salt, trioctylmethyl ammonium salt and diethylcyclohexadodecyl ammonium salt; acidic phosphoric acid ester and the like. These may be used alone or in combination of two or more thereof.

As the reaction catalyst, a catalyst used in the polymerization reaction of the polythiourethane resin may be used. For example, dialkyltin halide catalysts, such as dibutyltin dichloride and dimethyltin dichloride; dialkyltin dicarboxylate catalysts such as dimethyltin diacetate, dibutyltin dioctanoate, and dibutyltin dilaurate; dialkyltin dialkoxide catalysts such as dibutyltin dibutoxide and dioctyltin dibutoxide; dialkyltin dithio alkoxide catalysts such as dibutyltin di(thiobutoxide); dialkyltin oxide catalysts such as di(2-ethylhexyl)tin oxide, dioctyltin oxide, and bis(butoxydibutyltin)oxide; dialkyltin sulfide catalysts, and the like may be used. These may be used alone or in combination of two or more thereof.

As examples of the ultraviolet absorber, benzophenone-based, benzotriazole-based, salicylate-based, cyanoacrylate-based, oxanilide-based compounds, and the like may be used. As examples of the thermal stabilizer, metal fatty acid salt-based, phosphorus-based, lead-based, organotin-based compounds, and the like may be used. These may be used alone or in combination of two or more thereof.

The bluing agent may be included as a color control agent of the optical material prepared from the polythiourethane resin. For example, the bluing agent may have an absorption band in a wavelength band from orange to yellow in a visible light region.

Examples of the bluing agent may include a dye, a fluorescent whitening agent, a fluorescent pigment, an inorganic pigment, and the like, and may be appropriately selected according to physical properties or resin color required for the optical product to be manufactured. When a dye is used as the bluing agent, for example, a dye having a maximum absorption wavelength of 520nm to 600 nm, and preferably 540nm to 580 nm may be used. Preferably, anthraquinone-based dyes may be used.

A polythiourethane resin may be produced through a polymerization reaction of the polythiol-based compound included in the polythiol composition and the isocyanate-based compound.

In some embodiments, based on a total weight of the polymerizable composition for an optical material, the polythiol-based compound may be included in a content of about 40 wt.% to 60 wt.% and the isocyanate-based compound may be included in a content of about 40 wt.% to 60 wt.%, while the additive may be included in a content of about 0.01 wt.% to 1 wt.%.

According to one aspect of the present application, an optical product manufactured through the above-described optical composition may be provided.

For example, after degassing the polymerizable composition under reduced pressure, the resultant composition may be injected into a mold for molding an optical material. Mold injection may be performed, for example, in a temperature

range of 20 °C to 40 °C, and preferably 20°C to 35 °C.

After the mold injection, the temperature may be gradually increased, thereby allowing a polymerization reaction of the polythiourethane resin to proceed. The polymerization temperature may range from 20 °C to 150 °C, and preferably 25 °C to 125 °C.

After completion of polymerization, the polymerized polythiourethane resin may be separated from the mold to obtain an optical product. The optical product may be manufactured in the form of a spectacle lens, a camera lens, a light emitting diode, etc. according to a shape of the mold.

The refractive index of the optical product may be adjusted according to the type and/or content ratio of the polythiol-based compound and the isocyanate-based compound used in the optical composition, for example, it may be adjusted in a range of 1.65 to 1.75.

The optical product may be improved by adding surface treatment such as antifouling, color imparting, hard coat, surface polishing, hardness strengthening and the like.

According to the above-described embodiments, a metal sulfide having an absorbance within the above-described range may be used in synthesizing the polythiol-based compound. Accordingly, for example, the purity and yield of the tetrafunctional polythiol compound may be improved. Further, an optical product with inhibited optical defects such as white turbidity, yellowing, etc. may be obtained from the polythiol-based compound.

Hereinafter, embodiments provided in the present application will be further described with reference to specific experimental examples. However, the following experimental examples only illustrate the present invention and are not intended to limit the appended claims, and those skilled in the art will obviously understand that various alterations and modifications are possible within the scope and spirit of the present invention. Such alterations and modifications are duly included in the appended claims.

## Example 1

### 1) Synthesis of tetrafunctional polythiol-based compound

After introducing 60.0 wt. parts of water, 0.3 wt. parts of triethylamine and 73.0 wt. parts of 2-mercaptoethanol into a reactor, a temperature of the reactor was lowered to 0 °C, and 88.2 wt. parts of epichlorohydrin was slowly added dropwise at a temperature of 15 °C or lower, and then further stirred at 30 °C for 3 hours. Then, 145.8 wt. parts of sodium sulfide solution prepared of $Na_2S$, which had an absorbance of 0.75 when the absorbance was measured for light at a wavelength of 350 nm at 25 °C after dissolving the same in distilled water in an amount of 17.3 wt. parts based on 100 wt. parts of distilled water, was slowly added dropwise at 20 °C to 25 °C, followed by stirring for additional 3 hours.

Then, 473.2 wt. parts of 36% hydrochloric acid and 177.8 wt. parts of thiourea were introduced, and stirred for 3 hours while refluxing at 110 °C, such that a thiuronium salt reaction was proceeded.

After the obtained reaction solution was cooled to 50 °C, 305.6 wt. parts of toluene and 332.6 wt. parts of 50% NaOH were added, and then hydrolysis was conducted at 40 °C to 60 °C for 3 hours.

Then, the water layer was discarded after performing layer separation for 1 hour, and 120 wt. part of 36% hydrochloric acid was added to the obtained toluene solution, followed by acid washing once at 33 °C to 40 °C for 30 minutes. After acid washing, 250 wt. parts of degassed water (dissolved oxygen concentration of 2 ppm) was added, and washing was conducted 4 times for 30 minutes at 35°C to 45 °C. After removing toluene and residual moisture under heating and reduced pressure, it was filtered under reduced pressure through a PTFE type membrane filter thus to obtain 140 wt. parts of the tetrafunctional polythiol compound represented by the above Formula 1-1.

### 3) Preparation of optical composition and manufacturing of lens

After uniformly admixing 49.3 wt. parts of the polythiol compound prepared as described above, 50.7 wt. parts of xylene diisocyanate, 0.01 wt. parts of dibutyltin chloride and 0.1 wt. parts of phosphoric acid ester release agent produced by ZELEC® UN Stepan, a defoaming process was conducted at 600 Pa for 1 hour to prepare a polymerizable composition for an optical material.

Then, the composition filtered through a 3 μm Teflon filter was injected into a mold provided with a glass mold and a tape. A temperature of the mold was slowly increased from 25 °C to 120 °C at a rate of 5°C/min, and polymerization was performed at 120 °C for 18 hours. After the polymerization was completed, the mold was separated, followed by further curing the product at 120 °C for 4 hours to manufacture a lens sample.

## Example 2-8 and Comparative Examples

A tetrafunctional polythiol compound and a lens sample were prepared in the same manner as in Example 1, except that the absorbance of $Na_2S$ used in the synthesis of the polythiol-based compound was altered as shown in

Table 1 below.

In Examples 5 and 6, the purchased $Na_2S$ was washed with ethanol at 0 °C and dried, and then used after measuring and confirming the absorbance thereof.

## Experimental Examples

(1) Measurement of $Na_2S$ absorbance

17.3 wt. parts of the obtained or purchased $Na_2S$ was dissolved in 100 wt. parts of degassed water containing less than 10 ppm of dissolved oxygen, and then placed in a quartz cell having an optical path length of 50 mm, followed by measuring an absorbance to a light at 350 nm wavelength using a spectrophotometer (Lambda-365, PerkinElmer).

(2) Content assay through HPLC analysis

In the polythiol compositions according to each of the examples and comparative examples, a peak region % of the polythiol compound included in the composition was measured through HPLC analysis performed under the following conditions, and the sub-polythiol compound ratio according to Equation 1 was calculated.

<HPLC analysis condition>

i) Equipment: Agilent 1260 Infinity □
ii) Column: ZORBAX Eclipse Plus C18, 5 μm 4.6 × 250 mm
iii) Mobile phase gradient: Acetonitrile (0.1% Formic Acid): Water (0.01M Ammonium Formate) = 35-100: 65-0
iv) Solvent: Acetonitrile (0.1% Formic Acid)
v) Wavelength: 230 nm
vi) Flow rate: 1.0 ml/min
vii) Injection amount: 20 μl
viii) Sample pretreatment: sample: solvent = 0.1 g: 10 g

Specific compounds corresponding to the peaks of the HPLC graph were identified through liquid chromatography-mass spectroscopy (LC-MS). Specific conditions for LC-MS analysis are as follows.

<LC-MS analysis condition>

LC conditions

i) Equipment: LC 30A System (Shimadzu)
ii) Column: YMC-Pack ODS-A 150 mm × 6 mm (S-5 μm, 12 nm)
iii) Mobile phase gradient: Solvent A: water, Solvent B: Acetonitrile
A:B (60:40) 30 minutes, n (0:100) 10 minutes
iv) Flow rate: 1 ml/min
v) Column temperature: 40 °C
vi) Detector: PDA (190 to 800 nm)
vii) Injection Volume: 10 μL

Mass detector conditions

i) Equipment: Q Exactive (Thermo Fisher Scientific)
ii) Ionization Method: ESI
iii) Scan Range: m/z 130 to 1,950
iv) Polarity: Positive & Negative

Specifically, the tetrafunctional polythiol compound corresponding to Formula 1-1 was measured in the HPLC analysis graph with a retention time (RT) in a range of 25.0 to 27.0 min, and the sub-polythiol compound corresponding

to Formula 2-1 had a retention time of 29.5 to 30.5 min, and the sub-polythiol compound corresponding to Formula 2-2 was measured in a retention time range of 31.0 to 32.5 min.

(3) Evaluation of thiol value (SHV)

About 0.1 g of the polythiol composition prepared in each of the examples and comparative examples was introduced into a beaker, and 25 mL of chloroform was added, followed by stirring the mixture for 10 minutes. Then, 10 mL of methyl alcohol MeOH was added and stirred again for 10 minutes, and then, the resultant solution was titrated with a 0.1N iodine standard solution, followed by measuring SHV according to Equation 1 below (theoretical value: 91.7).

[Equation 1] SHV (g/eq.) = Sample weight (g)/{0.1 × Amount of iodine consumed (L)}

(3) Liquid refractive index

For the polythiol compositions synthesized in the examples and comparative examples, the refractive index at 25°C was measured using a liquid refractometer (RA-600 (Kyoto Electronics)).

(4) GPC purity

For the polythiol compositions synthesized in the examples and comparative examples, the purity was measured through gel chromatography analysis performed under the following conditions using an APC system (Waters).

i) Column: Acquity APC XT Column 45A (4.6*150 mm) × 2
ii) Mobile phase: THF
iii) Flow rate: 0.5 mL/min
iv) Total driving time: 10 minutes
v) Injection volume: 10 μl
vi) Detector: RID 40 °C

(5) Evaluation of stria

As described above, a lens sample having a diameter of 75 mm and -4.00D was prepared using the polymerizable composition according to each of the examples and comparative examples. A light from a mercury lamp light source was transmitted through the prepared lens sample, and the transmitted light was projected on a white plate to determine the presence or absence of stria according to the presence or absence of contrast. Standards for evaluation are as follows.

◦: Stria not observed
x: Stria clearly observed visually

(6) Evaluation of white turbidity of the lens

For the lens samples of the examples and comparative examples prepared as described above, each sample was irradiated with right beams from a projector in a dark room, and it was visually confirmed whether the lens had haze or an opaque material.

Standards for evaluation are as follows.

◦: No haze
△: Partial haze observed
x: Haze clearly observed as a whole

(7) Measurement of color index (yellow index (YI))

For the lens samples of the examples and comparative examples, YI was measured respectively using a colorimeter (Shinko, Colormate). Specifically, a lens sample having a thickness of 9 mm and φ 75 mm was prepared and chromaticity coordinates x and y were measured. YI was calculated by Equation 2 below based on the measured

values of x and y.

[Equation 2]

$$YI = (234 \times x + 106 \times y + 106)/y$$

Evaluation results are shown together in Table 1 below

[TABLE 1]

| | $Na_2S$ Absorbance | Absorbance after ethane washing | Ratio in Equation 1 (%) | Physical properties of polythiol composition | | | Physical properties of lens | | |
|---|---|---|---|---|---|---|---|---|---|
| | | | | SHV (g/eq.) | Liquid refractive index | GPC purity (%) | Stria | White turbidity | YI (Yellow Index) |
| Example 1 | 0.75 | - | 4.8 | 96.5 | 1.6465 | 84 | ○ | ○ | 19 |
| Example 2 | 1.12 | - | 4.4 | 96.2 | 1.6459 | 84 | ○ | ○ | 20 |
| Example 3 | 1.45 | - | 3.8 | 96.2 | 1.6458 | 82 | ○ | ○ | 22 |
| Example 4 | 1.94 | - | 3.0 | 96.1 | 1.6468 | 82 | ○ | ○ | 23 |
| Example 5 | 0.68 | 0.83 | 4.6 | 96.5 | 1.6466 | 83 | ○ | ○ | 19 |
| Example 6 | 0.55 | 0.88 | 4.4 | 96.4 | 1.6468 | 83 | ○ | ○ | 19 |
| Example 7 | 1.96 | - | 2.0 | 96.0 | 1.6467 | 82 | ○ | ○ | 23 |
| Example 8 | 1.99 | - | 1.1 | 95.8 | 1.6466 | 81 | ○ | ○ | 23 |
| Comparative Example 1 | 0.68 | - | 5.2 | 98.2 | 1.6475 | 76 | ○ | × | 20 |
| Comparative Example 2 | 0.55 | - | 5.5 | 98.9 | 1.6479 | 74 | ○ | × | 20 |
| Comparative Example 3 | 2.07 | - | 0.8 | 97.2 | 1.6465 | 79 | ○ | △ | 26 |
| Comparative Example 4 | 2.51 | - | 0.7 | 97.3 | 1.6466 | 78 | ○ | △ | 28 |

Referring to Table 1, in examples in which $Na_2S$ with a predetermined absorbance range is used and the sub-polythiol compound ratio of Formula 1 is adjusted to 1% to 5%, high-purity polythiol compounds and lens products were obtained while preventing white turbidity and discoloration.

## Claims

1. A polythiol composition, comprising:

    a tetrafunctional polythiol compound; and
    a sub-polythiol compound including a compound represented by $C_{13}H_{28}S_9$ and a compound represented by $C_{15}H_{32}S_{10}$, wherein a ratio of the sub-polythiol compound represented by Equation 1 below ranges from 1% to 5%:

    [Equation 1]

    $$\text{Sub-polythiol compound ratio} = 100\% \times [(\text{Peak region (\%) of } C_{13}H_{28}S_9) + (\text{Peak region (\%) of } C_{15}H_{32}S_{10})]/(\text{Peak region of tetrafunctional polythiol compound (\%)})$$

    (In Equation 1, the peak region (%) is a peak area (%) of the compound measured through a high performance liquid chromatography (HPLC) analysis graph obtained at a wavelength of 230 nm).

2. The polythiol composition according to claim 1, wherein the tetrafunctional polythiol compound comprises at least one of tetrafunctional polythiol compounds represented by Formulae 1-1 to 1-3 below:

    [Formula 1-1]

    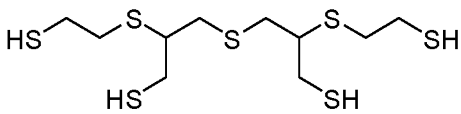

    [Formula 1-2]

    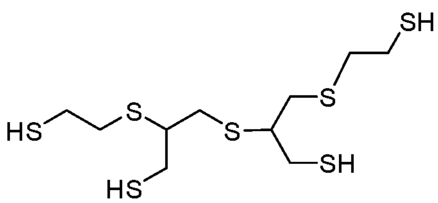

    [Formula 1-3]

    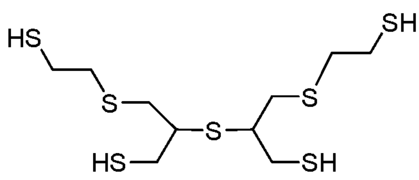

3. The polythiol composition according to claim 1, wherein the compound represented by $C_{13}H_{28}S_9$ has a structure of Formula 2-1 below:

[Formula 2-1]

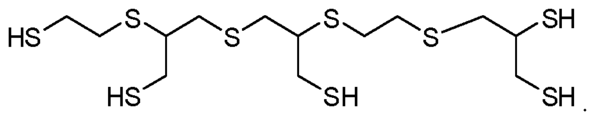

4. The polythiol composition according to claim 1, wherein the compound represented by $C_{15}H_{32}S_{10}$ has a structure of Formula 2-2 below:

[Formula 2-2]

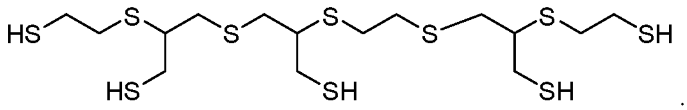

5. A method for preparation of a polythiol composition, the method comprising:

generating a polyol intermediate by introducing a metal sulfide to a preliminary polyol compound; and
converting the polyol intermediate into a polythiol-based compound through thiolation,
wherein the metal sulfide has an absorbance of 0.7 to 2.0 when measured for light at a wavelength of 350 nm in a quartz cell having an optical path length of 50 mm after dissolving the metal sulfide in distilled water in an amount of 17.3 parts by weight based on 100 parts by weight of distilled water.

6. The method according to claim 5, wherein the polythiol-based compound includes a tetrafunctional polythiol compound and a sub-polythiol compound having a greater molecular weight or a greater functional number than the tetrafunctional polythiol compound.

7. The method according to claim 6, wherein the sub-polythiol compound includes a compound represented by $C_{13}H_{28}S_9$ and a compound represented by $C_{15}H_{32}S_{10}$, wherein a ratio of the sub-polythiol compound represented by Equation 1 below ranges from 1% to 5%:

[Equation 1]

$$\text{Sub-polythiol compound ratio} = 100\% \times [(\text{Peak region (\%) of } C_{13}H_{28}S_9) + (\text{Peak region (\%) of } C_{15}H_{32}S_{10})]/(\text{Peak region of tetrafunctional polythiol compound (\%)})$$

(In Equation 1, the peak region (%) is a peak area (%) of the compound measured through a high performance liquid chromatography (HPLC) analysis graph obtained at a wavelength of 230 nm).

8. The method according to claim 5, further comprising, if the absorbance of the metal sulfide is less than 0.7, washing the metal sulfide with alcohol, water or an aqueous alcohol solution and then drying the same to adjust the absorbance of the metal sulfide in a range of 0.7 to 2.0

9. The method according to claim 5, wherein the metal sulfide includes $Na_2S$.

10. The method according to claim 5, wherein the absorbance of the metal sulfide ranges from 0.75 to 2.0.

11. An optical composition, comprising:

a polythiol composition which comprises a tetrafunctional polythiol compound, and a sub-polythiol compound including a compound represented by $C_{13}H_{28}S_9$ and a compound represented by $C_{15}H_{32}S_{10}$, wherein a ratio of the sub-polythiol compound represented by Equation 1 below ranges from 1% to 5%; and
an isocyanate-based compound:

[Equation 1]

$$\text{Sub-polythiol compound ratio} = 100\% \times [(\text{Peak region (\%) of } C_{13}H_{28}S_9) + (\text{Peak region (\%) of } C_{15}H_{32}S_{10})]/(\text{Peak region of tetrafunctional polythiol compound (\%)})$$

(In Equation 1, the peak region (%) is a peak area (%) of the compound measured through a high performance liquid chromatography (HPLC) analysis graph obtained at a wavelength of 230 nm).

**12.** A method for preparation of an optical composition, comprising:

preparing a polythiol-based compound; and
admixing the polythiol-based compound with an isocyanate-based compound,
wherein the step of preparing the polythiol-based compound includes:

generating a polyol intermediate by introducing a metal sulfide to a preliminary polyol compound; and
converting the polyol intermediate into a polythiol-based compound through thiolation,
wherein the metal sulfide has an absorbance of 0.7 to 2.0 when measured for light at a wavelength of 350 nm in a quartz cell having an optical path length of 50 mm after dissolving the metal sulfide in distilled water in an amount of 17.3 parts by weight based on 100 parts by weight of distilled water.

**13.** An optical product, comprising a copolymer of a polythiol composition and an isocyanate-based compound,

wherein the polythiol composition comprises a tetrafunctional polythiol compound, and a sub-polythiol compound including a compound represented by $C_{13}H_{28}S_9$ and a compound represented by $C_{15}H_{32}S_{10}$, and wherein a ratio of the sub-polythiol compound represented by Equation 1 below ranges from 1% to 5%:

[Equation 1]

$$\text{Sub-polythiol compound ratio} = 100\% \times [(\text{Peak region (\%) of } C_{13}H_{28}S_9) + (\text{Peak region (\%) of } C_{15}H_{32}S_{10})]/(\text{Peak region of tetrafunctional polythiol compound (\%)})$$

(In Equation 1, the peak region (%) is a peak area (%) of the compound measured through a high performance liquid chromatography (HPLC) analysis graph obtained at a wavelength of 230 nm).

**14.** The optical product according to claim 13, further comprising at least one additive selected from the group consisting of a release agent, a reaction catalyst, a thermal stabilizer, an ultraviolet absorber and a bluing agent.

**15.** A metal sulfide for synthesizing a polythiol-based compound, which has an absorbance of 0.7 to 2.0 when measured for light at a wavelength of 350 nm in a quartz cell having an optical path length of 50 mm after dissolving the same in distilled water in an amount of 17.3 parts by weight based on 100 parts by weight of distilled water.

## INTERNATIONAL SEARCH REPORT

International application No. **PCT/KR2021/011694**

**A. CLASSIFICATION OF SUBJECT MATTER**

**C08G 18/38**(2006.01)i; **C08G 18/70**(2006.01)i; **C07C 319/02**(2006.01)i; **C08L 75/04**(2006.01)i; **G02B 1/04**(2006.01)i; **C07C 319/20**(2006.01)i; **C07C 319/28**(2006.01)i; **C07C 321/14**(2006.01)i; **C07C 321/04**(2006.01)i

According to International Patent Classification (IPC) or to both national classification and IPC

**B. FIELDS SEARCHED**

Minimum documentation searched (classification system followed by classification symbols)

C08G 18/38(2006.01); B29D 11/00(2006.01); C07C 319/20(2006.01); C07C 319/22(2006.01); C07C 321/14(2006.01); C07D 301/27(2006.01); C07D 303/34(2006.01); C08L 75/12(2006.01); C08L 81/02(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal), STN (Registry, Caplus), Pubchem, google & keywords: 폴리티올 (polythiol), 광학 조성물 (optical composition), 금속황화물 (metallic sulfide), 흡광도 (absorbance)

**C. DOCUMENTS CONSIDERED TO BE RELEVANT**

| Category* | Citation of document, with indication, where appropriate, of the relevant passages | Relevant to claim No. |
|---|---|---|
| X | KR 10-2019-0122729 A (MITSUI CHEMICALS, INC.) 30 October 2019 (2019-10-30)<br>See paragraphs [0029]-[0043], [0074]-[0076], [0198]-[0201] and [0204]-[0207]; comparative examples 2 and 3; and claims 1, 3-5, 9 and 14. | 1-15 |
| A | KR 10-2011-0021371 A (JANG, Dong Gyu) 04 March 2011 (2011-03-04)<br>See entire document. | 1-15 |
| A | WO 2014-027428 A1 (MITSUI CHEMICALS, INC.) 20 February 2014 (2014-02-20)<br>See entire document. | 1-15 |
| A | KR 10-2018-0024561 A (SKC CO., LTD.) 08 March 2018 (2018-03-08)<br>See entire document. | 1-15 |
| A | KR 10-2015-0035489 A (MITSUBISHI GAS CHEMICAL COMPANY, INC.) 06 April 2015 (2015-04-06)<br>See entire document. | 1-15 |

☐ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **06 December 2021** | **06 December 2021** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2019)

**INTERNATIONAL SEARCH REPORT**
**Information on patent family members**

International application No.
**PCT/KR2021/011694**

| Patent document cited in search report | Publication date (day/month/year) | Patent family member(s) | Publication date (day/month/year) |
|---|---|---|---|
| KR 10-2019-0122729 A | 30 October 2019 | CN 110446696 A | 12 November 2019 |
| | | EP 3604279 A1 | 05 February 2020 |
| | | EP 3604279 A4 | 23 December 2020 |
| | | JP 6846507 B2 | 24 March 2021 |
| | | KR 10-2236080 B1 | 02 April 2021 |
| | | WO 2018-173820 A1 | 27 September 2018 |
| KR 10-2011-0021371 A | 04 March 2011 | KR 10-1594407 B1 | 17 February 2016 |
| WO 2014-027428 A1 | 20 February 2014 | BR 112014030076 A2 | 27 June 2017 |
| | | BR 112015000373 A2 | 27 June 2017 |
| | | CN 104321306 A | 28 January 2015 |
| | | CN 104321306 B | 19 October 2016 |
| | | CN 104321307 A | 28 January 2015 |
| | | CN 104321307 B | 17 August 2016 |
| | | CN 105906773 A | 31 August 2016 |
| | | CN 105906773 B | 29 May 2018 |
| | | CN 106432664 A | 22 February 2017 |
| | | CN 106432664 B | 28 May 2019 |
| | | EP 2845847 A1 | 11 March 2015 |
| | | EP 2845847 A4 | 29 April 2015 |
| | | EP 2845847 B1 | 31 August 2016 |
| | | EP 2845848 A1 | 11 March 2015 |
| | | EP 2845848 A4 | 20 May 2015 |
| | | EP 2845848 B1 | 24 February 2016 |
| | | EP 3026041 A1 | 01 June 2016 |
| | | EP 3026041 B1 | 19 April 2017 |
| | | EP 3093282 A1 | 16 November 2016 |
| | | EP 3093282 B1 | 10 May 2017 |
| | | IN 9746DEN2014 A | 31 July 2015 |
| | | IN 9747DEN2014 A | 31 July 2015 |
| | | JP 5319037 B1 | 16 October 2013 |
| | | KR 10-1661835 B1 | 30 September 2016 |
| | | KR 10-1879962 B1 | 18 July 2018 |
| | | KR 10-2014-0141723 A | 10 December 2014 |
| | | KR 10-2014-0142375 A | 11 December 2014 |
| | | KR 10-2016-0028486 A | 11 March 2016 |
| | | KR 10-2016-0028487 A | 11 March 2016 |
| | | US 2015-0126781 A1 | 07 May 2015 |
| | | US 2015-0133692 A1 | 14 May 2015 |
| | | US 2016-0017085 A1 | 21 January 2016 |
| | | US 2016-0024242 A1 | 28 January 2016 |
| | | US 9181179 B2 | 10 November 2015 |
| | | US 9181180 B2 | 10 November 2015 |
| | | US 9605105 B2 | 28 March 2017 |
| | | US 9637584 B2 | 02 May 2017 |
| | | WO 2014-027427 A1 | 20 February 2014 |
| KR 10-2018-0024561 A | 08 March 2018 | KR 10-1885879 B1 | 07 August 2018 |
| | | TW 201811741 A | 01 April 2018 |
| | | TW I646073 B | 01 January 2019 |
| | | WO 2018-043896 A1 | 08 March 2018 |
| KR 10-2015-0035489 A | 06 April 2015 | BR 112014024437 A2 | 25 July 2017 |

Form PCT/ISA/210 (patent family annex) (July 2019)

**INTERNATIONAL SEARCH REPORT**
**Information on patent family members**

International application No.
**PCT/KR2021/011694**

| Patent document cited in search report | Publication date (day/month/year) | Patent family member(s) | | | Publication date (day/month/year) |
|---|---|---|---|---|---|
| | | BR | 112014024437 | B1 | 03 November 2020 |
| | | BR | 112014024437 | B8 | 17 November 2020 |
| | | CN | 104379569 | A | 25 February 2015 |
| | | CN | 104379569 | B | 24 May 2017 |
| | | EP | 2865673 | A1 | 29 April 2015 |
| | | EP | 2865673 | A4 | 24 February 2016 |
| | | EP | 2865673 | B1 | 27 February 2019 |
| | | IN | 47DEN2015 | A | 22 May 2015 |
| | | JP | 6128124 | B2 | 17 May 2017 |
| | | KR | 10-2013700 | B1 | 23 August 2019 |
| | | TW | 201412726 | A | 01 April 2014 |
| | | TW | I570112 | B | 11 February 2017 |
| | | US | 2015-0158836 | A1 | 11 June 2015 |
| | | US | 9133151 | B2 | 15 September 2015 |
| | | WO | 2014-002876 | A1 | 03 January 2014 |

Form PCT/ISA/210 (patent family annex) (July 2019)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- KR 1020200111005
- KR 1020200165577
- KR 101338568